# EUROPÄISCHE PATENTSCHRIFT

(11) **EP 0 871 769 B1**
(45) Veröffentlichungstag und Bekanntmachung des Hinweises auf die Patenterteilung: **12.09.2001**
(21) Anmeldenummer: 96910034.6
(22) Anmeldetag: 10.04.1996
(51) Int. Cl.: C12Q 1/68, C12Q 1/44, C12P 19/34

(54) **VERFAHREN ZUR REDUZIERUNG DER BILDUNG VON ARTEFAKTEN WÄHREND TRANSKRIPTION VON RIBONUKLEINSÄUREN IN DESOXYRIBONUKLEINSÄUREN**
PROCESS FOR REDUCING THE FORMATION OF ARTEFACTS DURING TRANSCRIPTION OF RIBONUCLEIC ACIDS TO DEOXYRIBONUCLEIC ACIDS
PROCEDE PERMETTANT DE REDUIRE LA FORMATION D'ARTEFACTS LORS DE LA TRANSCRIPTION D'ACIDES RIBONUCLEIQUES EN ACIDES DESOXYRIBONUCLEIQUES

(30) Priorität: 11.04.1995 DE 19513728
(43) Veröffentlichungstag der Anmeldung: 21.10.1998
(73) Patentinhaber: Roche Diagnostics GmbH, 68298 Mannheim (DE)
(72) Erfinder: BAUER, Peter, D-12047 Berlin (DE); ROLFS, Arndt, D-10999 Berlin (DE); REGITZ-ZAGROSEK, Vera, D-10719 Berlin (DE); FLECK, Eckart, D-14163 Berlin (DE)
(86) Internationale Anmeldenummer: EP9601524
(87) Internationale Veröffentlichungsnummer: WO9632501

(56) Entgegenhaltungen:
- EP-A- 0 569 272
- EP-A- 0 632 134
- WO-A-94/12657
- NUCLEIC ACIDS RESEARCH, Bd. 25, Nr. 20, Oktober 1990, Seite 6149 XP000159871 DERAGON J-M ET AL: "Use of gamma radiation to eliminate DNA contamination for PCR"

### SEQUENZPROTOKOLL

(1) ALLGEMEINE ANGABEN:
   (i) ANMELDER:
      (A) NAME: Boehringer Mannheim GmbH
      (B) STRASSE: Sandhofer Strasse 116
      (C) ORT: Mannheim
      (E) LAND: Germany
      (F) POSTLEITZAHL: 68298
      (G) TELEFON: 0621/759 4348
      (H) TELEFAX: 0621/759 4457
   (ii) BEZEICHNUNG DER ERFINDUNG: Verfahren zur Reduzierung der Bildung von Artefakten
   (iii) ANZAHL DER SEQUENZEN: 6
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 20 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "oligonucleotide"
   (xi) SEQUENZBESCHREIBUNG: SEQ ID NO: 1:
   GGTATGGATG AGGACCTGGA 20
(2) ANGABEN ZU SEQ ID NO: 2:
   (i) SEQUENZKENNZEICHEN:
      (A) LÄNGE: 20 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonucleotide"
   (xi) SEQUENZBESCHREIBUNG: SEQ ID NO: 2:
   CTTCCACAGC CCTCGACTAA 20
(2) ANGABEN ZU SEQ ID NO: 3:
   (i) SEQUENZKENNZEICHEN:
      (A) LÄNGE: 20 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonucleotide"
   (xi) SEQUENZBESCHREIBUNG: SEQ ID NO: 3:
   CCTTCGACGC ACAATGCTTG 20
(2) ANGABEN ZU SEQ ID NO: 4:
   (i) SEQUENZKENNZEICHEN:
      (A) LÄNGE: 20 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonucleotide"
   (xi) SEQUENZBESCHREIBUNG: SEQ ID NO: 4:
   ATGATTGTCC CAAAGCTGGA 20
(2) ANGABEN ZU SEQ ID NO: 5:
   (i) SEQUENZKENNZEICHEN:
      (A) LÄNGE: 20 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonucleotide"
   (xi) SEQUENZBESCHREIBUNG: SEQ ID NO: 5:
   TGCTTGGAGA AGAAGTTGCC 20
(2) ANGABEN ZU SEQ ID NO: 6:
   (i) SEQUENZKENNZEICHEN:
      (A) LÄNGE: 20 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligonucleotide"
   (xi) SEQUENZBESCHREIBUNG: SEQ ID NO: 6:
   TCTGGGGGTA ACAGTGACCT 20

## Beschreibung

Gegenstand der Erfindung sind ein Verfahren zur Transkription von Ribonukleinsäuren (RNS) einer Probe zu Desoxyribonukleinsäuren (DNS), ein Verfahren zur Herstellung einer Vielzahl von DNS-Molekülen unter Verwendung von RNS-Molekülen sowie Reagenzien zur Reduzierung von Artefakten und Reagenzkits zur Durchführung der Verfahren.

Analyseverfahren für Nukleinsäuren können sowohl von RNS als auch von DNS ausgehen. Beide Möglichkeiten haben Vorteile. Bei Verfahren, die RNS als Ausgangsmaterial benutzen, wird in vielen Fällen zunächst die Ribonukleinsäure in Desoxyribonukleinsäure umgeschrieben, transkribiert. Dies geschieht beispielsweise vor dem Hintergrund, daß Desoxyribonukleinsäuren (DNS) stabiler sind. Aus Ribonukleinsäuren (RNS) sind jedoch in vielen Fällen auch Informationen erhältlich, die bei Analyseverfahren, die von DNS ausgehen, nicht erhältlich sind. Dies liegt daran, daß die genomische DNS noch Intronsequenzen enthält, die bei der körpereigenen Prozessierung in RNS verloren gehen. Verfahren, bei denen DNS nachgewiesen wird, können daher, je nachdem, ob die betrachtete Sequenz Introns enthält oder nicht, zu denselben Analysewerten kommen oder nicht. In solchen Fällen, bei denen nur die RNS nachgewiesen werden soll, führt daher eine Verunreinigung durch DNS zu einer positiven Verfälschung des Analyseergebnisses, wenn keine Vorsorge getroffen wird. Eine Möglichkeit der Lösung dieses Problems ist die Verwendung von sogenannten junction-Primern. Junction-Primer sind Oligonukleotide, deren Sequenz so gewählt ist, daß ihr 5'-Ende komplementär zu der Sequenz des 5'-Endes eines Exons ist und ihr 3'-Ende einige Nukleotide enthält, die zu den Nukleotiden des 3'-Endes des benachbarten Exons komplementär sind, nicht jedoch zu den Nukleotiden am 3'-Ende des im Genom dazwischen liegenden Introns. In einem solchen Fall findet zumindest teilweise eine Vermeidung der Verfälschung des Ergebnisses durch verunreinigende DNS statt.

Eine solche Unterscheidung zwischen DNS und RNS ist jedoch nicht möglich, wenn Transkripte von intronfreien Gen untersucht werden sollen. Darüber hinaus stellen Pseudogene dasselbe Problem dar, da sie identisch in der Länge sind und somit nicht von den Transkripten der RNS unterschieden werden können. In diesen Fällen hat es sich somit als zweckmäßig erwiesen, die mRNS-Probe mit RNasefreier DNase vor Durchführung der Transkriptionsreaktion zu behandeln. Ein solches Verfahren ist beispielsweise beschrieben in BioTechniques 9: 262 - 268 (1990) und BioTechniques 13: 726 - 729 (1992).

In BioTechniques 13: 726-729 (1992) sind insbesondere zwei Verfahren zur Entfernung von kontaminierender DNA in einer Probe für die anschließende RT-PCR beschrieben. Im ersten Verfahren wird inmobilisierte DNase I in Anwesenheit von Mn²⁺-Ionen verwendet; im zweiten, alternativ zu verwendenden Verfahren - welches als Standard-DNase I-Behandlung gilt - wird eine Lösung von DNase I in Anwesenheit von Mg²⁺-Ionen eingesetzt.

Die Bewertung im Falle intronhaltiger Genabschnitte eines effektiven DNS-Abbaus erfordert bisher eine Polymerasekettenreaktion eines Teils der DNase behandelten, jedoch noch nicht reverse transkribierten RNS mit einem Primerset, welches eine Größendiskriminierung zwischen PCR-Produkten, die einerseits von der cDNS und andererseits von der genomischen DNS herrühren, erlaubt.

DNase I erfordert divalente Metallionen (J. Biol. Chem. 243: 4409 - 4416 (1968)) als Cofaktor. Es ist bekannt, daß das Optimum der Konzentration an Mg²⁺-Ionen ungefähr eine Größenordnung größer ist als das von Mn²⁺. Darüber hinaus ist bekannt, daß eine Erhöhung der Mn²⁺-Konzentration nicht wie für Mg²⁺ oder Ca²⁺ die DNase-Reaktion inhibiert.

Kern der vorliegenden Erfindung ist die Erkenntnis, daß bei Einsatz von Mn²⁺-Ionen die Menge an Artefakt-DNS, welche durch replikative Amplifizierung, z. B. mittels der Polymerasekettenreaktion (PCR), gebildet wird, deutlich reduziert bzw. praktisch vollständig eliminiert ist. Darüber hinaus ist der DNase-Verdau mit Mn²⁺-Ionen wesentlich effektiver als mit Mg²⁺.

Gegenstand der Erfindung ist daher ein Verfahren zur Transkription von Ribonukleinsäuren einer Probe zu Desoxyribonukleinsäuren, enthaltend die Schritte:
a) Behandlung der ribonukleinsäurehaltigen Probe mit einer Lösung von DNase I und anschließend
b) Transkription der Ribonukleinsäuren in Desoxyribonukleinsäuren,
wobei Schritt a) in Anwesenheit von Mn²⁺-Ionen durchgeführt wird.

Ebenfalls Gegenstand der Erfindung ist ein Verfahren zur Herstellung einer Vielzahl von DNS-Molekülen unter Verwendung von RNS-Molekülen, Reagenzkits zur Verwendung in diesen Verfahren sowie die Verwendung einer lösung von DNase I und Mn²⁺-Ionen zur Reduktion der Bildung von Artefakt-DNS bei Transkriptionsreaktionen.

Als Ribonukleinsäuren nach der vorliegenden Erfindung können beliebige Ribonukleinsäuren benutzt werden, z. B. mRNS, tRNS, rRNS. Besonders zweckmäßig ist die Erfindung bei intronloser mRNS und mRNS, die als Pseudogen vorkommt oder von der nicht bekannt ist, ob Pseudogene vorliegen.

Die Ribonukleinsäuren können nach der vorliegenden Erfindung in stark mit korrespondierender DNS verunreinigter Form vorliegen. Dies ist einer der Hauptvorteile der Erfindung. Aus diesem Grund können bereits weniger stark aufgereinigte RNS-haltige Proben in das erfindungsgemäße Verfahren eingesetzt werden. Insbesondere kann der Gehalt an Desoxyribonukleinsäuren - sei es zu der entsprechenden Ribonukleinsäure korrespondierenden oder auch fremden Desoxyribonukleinsäuren - höher sein als bei den Proben, die nach Verfahren des Standes der Technik zur Transkription der Ribonukleinsäuren eingesetzt wurden. Die Probe kann einem beliebigen zu untersuchenden Objekt entnommen sein. Es kann sich z. B. um ein Lysat von Gewebe oder Einzelzellen handeln. Die Herstellung ribonukleinsäurehaltiger Proben, insbesondere von Lysaten, ist dem Fachmann bekannt. Die Ribonukleinsäuren können in der Probe gelöst vorliegen, sie können jedoch auch in an eine feste Phase immobilisierter Form vorliegen. Wichtig für die Verfahren der Transkription von Ribonukleinsäuren ist letztendlich, daß die Probe mit einem Enzym zum Abbau von DNS umgesetzt werden kann.

In einem ersten Schritt wird die ribonukleinsäurehaltige Probe mit einer Lösung von DNase I in Anwesenheit von Mn²⁺-Ionen behandelt. Entsprechende DNS abbauende Enzyme wie DNase I sind dem Fachmann bekannt. DNase I ist eine Endonuklease, die doppelsträngige oder einzelsträngige DNS unter Bildung eines komplexen Gemisches von Mono- und Oligonukleotiden mit 5'-Phosphoryl-Enden hydrolysiert. Die Bedingungen zur Behandlung der Probe mit der Mn²⁺-innenhaltigen Lösung unterscheiden sich mit Ausnahme der Anwesenheit der Mn²⁺-Innen nicht wesentlich von den Bedingungen, bei denen die Behandlung von ribonukleinsäurehaltigen Proben mit Mg²⁺-ionenhaltigen DNase-Lösungen bekannt sind. Die erfindungsgemäß vorhandenen Mn²⁺-Ionen sind bevorzugt in einer Konzentration von mehr als 10⁻⁶ M vorhanden. Bevorzugt liegt die Konzentration an Mn²⁺ zwischen 10⁻⁵ und 10⁻¹ M, besonders bevorzugt zwischen 10⁻⁴ und 10⁻² M. Da es als möglich betrachtet wird, daß die DNA assoziierte Artefakt-Bildung auf die Anwesenheit der Mg²⁺-Ionen zurückzuführen ist, sollte der Einsatz von Mg²⁺ in der Verdauungslösung vermieden werden.

Zur Durchführung des DNS-Abbauschrittes wird der zu behandelnden Probe bevorzugt eine Lösung zugegeben, welche die erforderlichen Reagenzien in einer Konzentration enthält, bei der der Abbau in dem entstehenden Reaktionsgemisch stattfinden kann. Anschließend wird für eine ausreichende Zeit inkubiert. Bevorzugt sind Inkubationsdauern zwischen 5 und 60 Minuten bei Temperaturen zwischen 36. und 38 °C.

Bevorzugt wird anschließend das DNS abbauende Enzym auf bekannte Weise, z. B. durch Hitze (5 Minuten, 90 °C) oder, bevorzugt, Extraktion, inaktiviert. Dies geschieht, damit die in der nachfolgenden Reaktion gebildeten Desoxyribonukleinsäuren nicht wieder abgebaut werden.

Danach findet die Transkription der Ribonukleinsäuren in Desoxyribonukleinsäuren statt Die Ribonukleinsäuren können hierbei entweder in dem im vorangehenden Schritt entstandenen Reaktionsgemisch belassen werden, sie können jedoch auch zwischengereinigt werden. Die Transkription der Ribonukleinsäuren kann auf bekannte Weise durchgeführt werden. Unter Transkription von Ribonukleinsäuren ist der dem Fachmann bekannte Vorgang der Bildung von DNS aus RNS unter Verwendung der RNS als Substrat für die enzymkatalysierte Kondensation von Monodesoxyribonukleotiden gemeint. Dieser Vorgang ist in Molecular Cloning: A Laboratory Manual, Cold Spring Harbour, NY, 1989 beschrieben. Bevorzugtes Enzym zur Transkription ist die reverse Transkriptase, insbesondere reverse Transkriptase aus MMLV, insbesondere Superscript (RNase H deletiert).

Außerdem Gegenstand der Erfindung ist ein Verfahren zur Herstellung einer Vielzahl von DNS-Molekülen unter Verwendung von RNS-Molekülen, enthaltend die Schritte:
a) Behandlung einer ribonukleinsäurehaltigen Probe mit einer Lösung von DNase I in Anwesenheit von Mn²⁺-Ionen,
b) Transkription der Ribonukleinsäuren in Desoxyribonukleinsäuren und
c) Vermehrung der Desoxyribonukleinsäuren.

Unter Vermehrung der Desoxyribonukleinsäuren wird die Herstellung einer Vielzahl von Kopien der DNS bzw. von Teilen davon verstanden. Verfahren zur Vermehrung von Desoxyribonukleinsäuren sind dem Fachmann bekannt. Ein prominenter Vertreter dieser Reaktionen ist die Polymerasekettenreaktion (PCR) nach EP-B-0 201 184. Bei dieser Reaktion wird der zu vermehrenden Desoxyribonukleinsäure ein Überschuß an Primer molekülen zugegeben, wobei ein Teil der Primer komplementär zu einem nicht das 5'-Ende bildenden Sequenzbereich und ein anderer Teil der Primer zum komplementären Strang komplementär ist, so daß das Verlängerungsprodukt jedes Primers wieder als Matrize für die Verlängerung des anderen Primers dienen kann. Die Verlängerung der Primer findet durch polymerasekatalysierte Anhängung von Mononukleotideinheiten an die Primer statt.

Als Primer können erfindungsgemäß auch junction-Primer verwendet werden. Bevorzugt ist der Teil, der zum 3'-Ende des zweiten Exons komplementär ist, 2 - 4 nt, besonders bevorzugt 3 nt lang, während der daran in 5'-Richtung des Primers anschließende Teil so lang ist, daß eine selektive Hybridisierung mit dem 5'-Ende des ersten Exons möglich ist, d. h. bevorzugt 10 - 30, besonders bevorzugt 15- 20 nt.

Es sind weitere Verfahren zur Vermehrung von Desoxyribonukleinsäuren bekannt, z. B. die Ligasekettenreaktion (LCR) nach EP-A-0 320 308, bei der jeweils 2 erste Nukleinsäuren, welche an benachbarte Bereiche der DNS hybridiseren, miteinander kovalent verbunden werden. Das Verknüpfungsprodukt der ersten beiden Nukleinsäuren wird als Matrize für die Verknüpfung zweier weiterer, benachbart an das Verknüpfungsprodukt anhybridisierender Nukleinsäuren benutzt. Auch hier findet durch Cyclusführung eine Amplifikation eines Sequenzbereiches statt.

Ebenfalls verwendet werden kann als Verfahren zur Vermehrung der Desoxyribonukleinsäuren das Verfahren nach WO 90/01069, welches sowohl Extensions- als auch Verknüpfungsreaktionen beinhaltet.

Eine weitere Möglichkeit der Vermehrung der DNS bietet das Verfahren nach EP-A-0 329 822, bei welchem die Vermehrungszyklen einen Schritt zur DNS-abhängigen RNS-Polymerasereaktion beinhalten.

All diesen bisher beschriebenen Vermehrungsverfahren ist gemeinsam, daß sie eine sequenzspezifische Vermehrung bewirken. Sofern nicht komplizierte Gegenmaßnahmen eingeleitet werden, und wenn nicht besondere Umstände vorliegen, würden diese Verfahren verunreinigende, im wesentlichen gleiche Sequenzen beinhaltende genomische DNS genauso vermehren wie die eigentlich für die Vermehrung zu verwendenden, aus den RNS-Molekülen durch Transkription hergestellten Desoxyribonukleinsäuren. Dies führt jedoch zu einem unerwünschten Hintergrundsignal, welches in der Regel größer ist als das Hintergrundsignal, welches durch die üblicherweise in nukleinsäurehaltigen Proben vorhandenen, nicht zu vermehrenden bzw. nachzuweisenden Nukleinsäuren anderer Sequenzen, hervorgerufen wird. Bei solchen Verfahren zur Herstellung einer Vielzahl von DNS-Molekülen ist die vorliegende Erfindung daher ganz besonders geeignet.

Ohne an diese Erklärung gebunden zu sein, weisen die bisherigen Ergebnisse der Versuche auf folgendes hin. Da nach Behandlung der DNS und RNS enthaltenden Probe mittels des DNS-abbauenden Enzyms keine DNS mehr nachzuweisen war (beispielsweise durch PCR mit geeigneten Primern), weist die Tatsache, daß für den Fall, daß die Abbaureaktion in Gegenwart von Magnesium-Ionen durchgeführt wurde, dennoch DNS-Signale festgestellt wurden, darauf hin, daß eine Artefakt-Konstruktion der offenbar nur teilweise verdauten DNS der Probe stattgefunden hat. Bei erfindungsgemäßem Einsatz von Mangan-Ionen kann offenbar eine solche Rekonstruktion nicht mehr stattfinden. Die Bildung von Artefakt-DNS in der nachfolgenden Transkriptionsreaktion wird dadurch vermieden.

Gegenstand der Erfindung ist weiterhin ein Reagenz zur Reduzierung der Bildung von Artefakt-DNS bei Transkriptionsreaktionen, welches eine Lösung von DNase I und Mn²⁺-Ionen enthält. Dieses Reagenz kann außerdem noch weitere Reagenzien, wie sie üblicherweise zur Einstellung von Bedingungen erforderlich sind, bei denen DNS in RNS-haltigen Proben abgebaut wird. Hierzu gehören insbesondere Puffer zur Einstellung des für die Abbaureaktion optimalen pH-Wertes.

Als Artefakt-DNS wird im Sinne der Erfindung DNS bezeichnet, die genomische Sequenzen enthält, jedoch durch eine in-vitro-Reaktion gebildet wurde, die nicht auf Transkription von RNS beruht.

Außerdem Gegenstand der Erfindung ist ein Reagenzkit zur Transkription von Ribonukleinsäuren in Desoxyribonukleinsäuren, enthaltend in getrennten Behältern eine Lösung von DNase I und Mn²⁺-Ionen sowie eine reverse Transkriptase. Dieser Reagenzkit enthält daher die für die Schritte a) und b) erforderlichen Reagenzien in getrennten Behältern. Für die reverse Transkriptionsreaktion sind neben reverser Transkriptase Mg²⁺-Ionen, Puffer, die zum Einbau erforderlichen Desoxyribonukleosidtriphosphate sowie RNase-Inhibitoren bevorzugt.

Ebenfalls Gegenstand der Erfindung ist ein Reagenzkit zur Herstellung einer Vielzahl von DNS-Molekülen aus RNS-Molekülen enthaltend in getrennten Behältern:
- eine Lösung von DNase I und Mn²⁺-Ionen,
- eine reverse Transkriptase und
- Reagenzien zur Vervielfältigung von DNS.

Die Reagenzien zur Vervielfältigung der DNS richten sich nach dem gewünschten Vermehrungsverfahren. Für die Durchführung der Polymerasekettenreaktion werden beispielsweise die auf den zu vermehrenden Bereich der DNS ausgerichteten Primer, eine bevorzugt thermostabile, DNS-abhängige DNS-Polymerase sowie die für die gewünschte Verlängerung erforderlichen Monodesoxyribonukleosidtriphosphate benötigt. Die Reagenzien hierfür sind beispielsweise in EP-B-0 201 184 beschrieben.

Die auf erfindungsgemäße Weise hergestellten Transkripte können zum Nachweis ursprünglich in einer Probe vorhandener RNS verwendet werden. Hierzu wird zunächst das erfindungsgemäße Verfahren zur Transkription durchgeführt, wobei das Verfahren durch Einsatz sequenzspezifischer Primer für bestimmte RNS-Sequenzen spezifisch gemacht werden kann. Nach einer gewünschtenfälls angeschlossenen Amplifikation der Transkript DNS, z. B. mit der PCR, können die gebildeten Nukleinsäuren, beovorzugt durch Hybridisierung mit zu den Nukleinsäuren komplementären Nukleinsäuresonden und Nachweis der Hybridisierung, auf bekannte Weise nachgewiesen werden. Das erfindungsgemäße Verfahren ist daher besonders geeignet auf dem Gebiet der Nukleinsäurediagnostik in der Molekularbiologie und der klinischen Labordiagnostik.

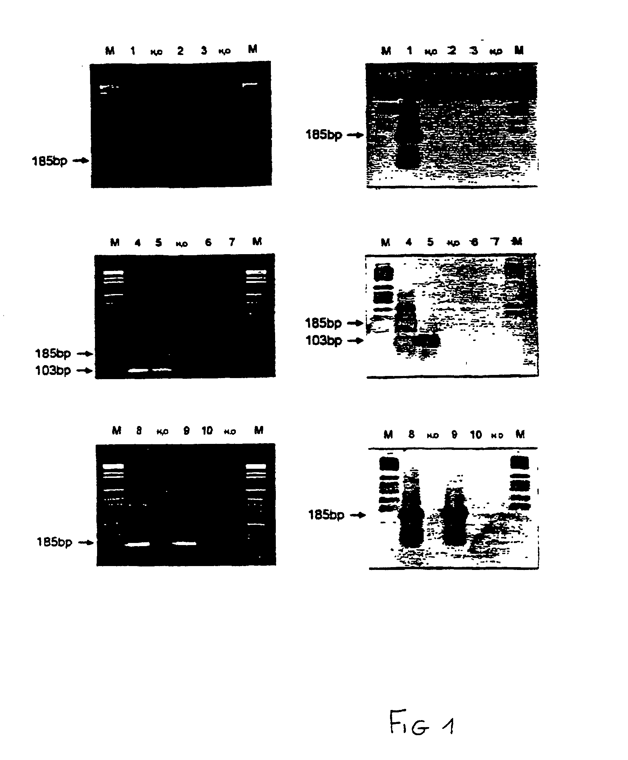

Figur 1 zeigt die Bildung von Transkripten unter verschiedensten Bedingungen, einerseits unter Einsatz von Mg²⁺, andererseits unter erfindungsgemäßem Einsatz von Mn²⁺.

Die folgenden Beispiele sollen die Erfindung näher erläutern.

### Beispiel 1

### RNS-Isolierung

RNS wurde aus tiefgefrorenen menschlichem Herzgewebe extrahiert. Die Proben wurden homogenisiert, die gesamte zelluläre RNS unter Verwendung der Säure Guanediniumthiocyanat/Phenol/Chloroformextraktionsmethode (Anal. Biochem. 162: 156-159 (1987)) extrahiert und bei 20.000 g, 4 °C mit 100 % Isopropanol präzipitiert. Die Pellets wurden mit 75 % Ethanol gewaschen, getrocknet und in DEPC-behandeltem Wasser resuspendiert. Alle RNS-Proben wurden mittels PCR getestet, um mögliche DNS-Kontaminationen aufzuzeigen (unter Verwendung des unten genannten Pyruvatdehydrogenasegen (PDH)-Primersystems).

### DNase I-Spaltung

1 µg RNS wurde mit 2 U DNase I (Boehringer Mannheim GmbH, BRD) für 10 Minuten bei 37 °C in Anwesenheit von 40 U RNasin/µg RNS (Promega, BRD) behandelt Die Endkonzentration von Mg²⁺ bzw. Mn²⁺ betrug 1 mM. Nach DNase I-Verdau wurde ein Aliquot auf 90 °C für 5 Minuten erhitzt.

### Reverse-Transkription

90 % der mit DNase I behandelten RNS-Probe wurde für die Reverse-Transkription (RT)-Reaktion verwendet. Der Rest wurde als PCR-Kontrolle eingesetzt. Bis zu 1 µg DNase I behandelte RNS wurde mit 500 ng Zufallshexanukleotiden (Random) für 10 Minuten bei 70 °C inkubiert und anschließend bei 4 °C aufbewahrt. Zum Start der Reaktion wurde ein Gemisch von 100 U Superscript MMLV reverse Transkriptase (Gibco-BRL, BRD), 3 mM MgCl₂, 50 mM Tris-HCL (pH 8.3), 75 mM KCl, 10 mM DTT, 0.5 mM dNTPs und 40 U RNasin zugegeben. Darin wurde die Probe 50 Minuten bei 42 °C inkubiert. Die Reaktion wurde anschließend 5 Minuten auf 95 °C erhitzt.

### Vermehrung der Desoxyribonukleinsäuren durch Polymerasekettenreaktion und Blot-Verfahren

Ein Intron überspannendes Primersystem für das Pyruvatdehydrogenasegen (PDH) mit dem Plus-Primer: und dem Minus-Primer wurde benutzt, um ein Amplikon der Länge 185 Basenpaare (bp) auf dem DNS-Niveau und 103 Basenpaare auf dem cDNS-Niveau zu erzeugen. In früheren Experimenten war demonstriert worden, daß für diese PDH-Genregion keine Pseudogene existieren. Zur Kreirung größerer Amplikons (538 bp) wurden für das intronlose Angiotensin II-Typ I-Rezeptorgen (ATR 1) spezifische Primer verwendet:

### Plus-Primer:

### Minus-Primer:

Die Reaktionen wurden durchgeführt mit 8 pMol jedes Primers, 50 mMKCl, 10 mM TrisxHCl (pH 8,3), 1.5 mM MgCl₂, 100 µM Desoxyribonukleosidtriphosphaten, 0.4 µM jedes Oligonukleotids, 1.0 U Taq-Polymerase (Perkin Eimer Cetus) und 50 ng Probennukleinsäure in einem Volumen von 25 µl. Zur Durchführung der Reaktion wurde ein Thermocycler 9600™ Perkin Eimer benutzt. Zunächst wurde die Probe bei 95 °C denaturiert. Anschließend wurde folgendes Temperaturprofil während 40 Zyklen durchgeführt: 45 sec. 95 °C, 60 sec. 60 °C, 45 sec. 72 °C und anschließend 5 Minuten bei 72 °C.

6 µl der Reaktionsmischung wurden einem 3 % Agarosegel (3 % 3:1 NuSieve™, FMC) mit anschließender Elektrophorese unterworfen. Das Gel wurde anschließend mit Denaturierungspuffer (1.5 M Natriumchlorid, 0.5 M Natriumhydroxid, für 10 Minuten) behandelt, 10 Minuten neutralisiert (pH 5,0) und auf eine Nylonmembran geblottet (positiv geladene Nylonmembran, Boehringer Mannheim GmbH). Der Neutralisierungs- und der Blottingpuffer bestanden aus 1.0 M TrisHCl, 2.0 M NaCl (pH 5.0). Die Membranen wurden 30 Minuten prähybridisiert in 5 xSSC-Hybridisierungspuffer (1 xSSC:0.12 M NaCl, 0,015 Natriumcitrat, 2.0 Gewichtsprozent Blockierungsreagenz (Boehringer Mannheim GmbH), 0.02 Gewichtsprozent SDS, 0.1 Gewichtsprozent N-Laurylsarkosin). Nach 6 Stunden Blotzeit wurde unmittelbar eine UV-Vernetzung mit 120 mJ (Stratalinker™, Stratagene) durchgeführt. Die Hybridisierung wurde für mindestens 6 Stunden bei 62 °C in 5 ml Hybridisierungslösung (enthaltend 2 µl mittels PCR synthetisierter, vorher denaturierter mit DIG-11-dUTP (Boehringer Mannheim GmbH, Bestell-Nr. 1093657) markierter (PCR: Clinical Diagnostics and Research, Springer Verlag, Berlin, S 121 und Herstellerangaben) Sonden in 5 x SSC; 0,1 % (v/v)N-Laurylsarkosin(Na-Salz); 0,02 %. (w/v) Natriumdodecylsulfat; 2 %. (w/v) Blocker-Reagenz (Boehringer Mannheim, Best.-Nr. 1096176, Casein-fraktion von entfetteter Trockenmilch) ausgeführt.

### Zur Herstellung der Sonden wurde folgendermaßen vorgegangen:

Bei Verwendung von Primerpaaren in der amplifizierten Region (Plus-Primer:
5'-TGCTTGGAGAAGAAGTTGCC-3', Minus-Primer: 5'-
TCTGGGGGTAACAGTGACCT-3') wurde ein 94 bp Amplikon direkt von der Kontroll-DNS amplifiziert. Dieses kurze Amplikon wurde nach der Ethidiumbromid-Methode in einem präparativen 1 %-NuSieve GTG Agarosegel untersucht. Der Elektrophoresepuffer setzt sich zusammen aus 0.04 M Tris-Acetat (pH 8.0). Das Amplikon wurde aus dem Gel ausgeschnitten und 5 µl der geschmolzenen Agarose wurden reamplifiziert, wobei die oben genannten Primer und ein dNTP Markierungsgemisch verwendet wurden, in dem 35 % der dTTP durch DIG-dUTP ersetzt waren. Diese Sonde wurde in den Hybridisierungsschritt ohne weitere Reinigung eingesetzt.

Nach 2maligem Waschen (5 Minuten, Raumtemperatur) in 2 xSSC-0.1% SDS und weiteren 2maligem Waschen (15 Minuten, 62 °C) in 0.2 xSSC-0.1 % SDS wurden die Membranen direkt in den chromogenen Detektionspuffer (0.1 mol/l Maleinsäure, 0.15 mol/l Natriumchlorid, pH 7.5, 0.3 Gewichtsprozent Tween-20) überführt.

Die Chromogen-Detektion wurde nach Rolfs et al., PCR: Clinical Diagnostics and Research, Springer Verlag, 1992, durchgeführt. Hierzu wurde die Membran mit Waschpuffer 1 bis 5 Minuten bei Raumtemperatur gewaschen und anschließend in Puffer 2 30 Minuten lang inkubiert. Danach wurde die Membran mit Antikörperkonjugatlösung 30 Minuten inkubiert. Anschließend wurde 2mal für 15 Minuten mit 100 ml Waschpuffer gewaschen. Danach wurden die Membranen 2 bis 5 Minuten in Puffer 3 behandelt. Danach wurden die Membranen im Dunkeln in einer Lösung von 55 µl NBT und 35 µl BCIP in 10 ml Puffer 3 für 6 Stunden inkubiert. Die Farbreaktion wurde für 5 Minuten mit Puffer 4 gestoppt. Anschließend wurde die Farbbildung detektiert.

Die Reagenzien für die Chromogen-Detektion wurden dem DIG-DNS-Labelling and Detection Kit non-radioactive (Boehringer Mannheim, Bestell-Nr. 1093657) entnommen und wie folgt geändert:

### Waschpuffer:

- Puffer:: 1 + 0,3 % (W/V) Tween™ 20
- Puffer 2:: Blockierungslösung 1 : 10 verdünnt in Puffer 1 (Endkonzentration 1 % Blockierungsreagenz)
- Puffer 3:: 0.1 M Tris HCl; 0.1 M NaCl; 50 mM MgCl₂; pH 9.5 (20 °C)
- Puffer 4:: 10 mM Tris-HCl; 1 mM EDTA; pH 8 (20 °C)
- NBT:: 75 mg/ml in 70 % Dimethylformamid (V/V)
- BCIP:: 50 mg/ml in Dimethylformamid

### Ergebnis

Obwohl die RNS-Proben mindestens 2mal mit Guanidiniumthiocyanat/Phenol/Chloroform extrahiert wurden, zeigten sich als klarer Beweis für verunreinigende DNS mehr oder weniger intensive PDH-spezifische Signale beim direkten Testen mit Hilfe der PCR, wenn keine weitere DNase I-Behandlung durchgeführt wurde (Figur 1, Spalte 1). Nach Behandlung eines Teils der RNS mit DNase I entweder mit Mg²⁺ oder Mn²⁺-Puffer wurde kein weiteres DNS-Signal (185 bp Fragment) in der PCR-Reaktion sichtbar (Figur 1, Spalten 2 und 3). Wenn DNase I/Mg²⁺-Puffer verdaute Reaktionsmischung für die reverse Transkriptasereaktion verwendet wurde, gefolgt von der PCR-Amplifikation, wurde das spezifische 103 bp cDNS-Fragment sichtbar, überraschenderweise jedoch auch das auf der Artefakt-DNS beruhende 185 bp Amplikon (Figur 1, Spalte 4). Im Gegensatz hierzu, wenn DNase-Verdau mit Mn²⁺-Puffer, gefolgt von reverser Transkriptase und PCR durchgeführt wurde, war nur das 103 bp cDNS-Amplikon sichtbar (Figur 1, Spalte 5). Die hohe Effizienz der Spaltung mit Hilfe von Mn²⁺ als Kofaktor für DNase I ist auch erkennbar, wenn 50 ng DNS eingesetzt werden bei 10 Minuten Reaktionszeit: Wenn beide Proben mittels PCR amplifiziert werden (ohne reverse Transkriptasereaktion), erzeugt nur der DNase/Mg²⁺-Puffer ein Amplikon (Figur 1, Spalte 9), während die DNase I/Mn²⁺ behandelte DNS vollständig verdaut wurde (Figur 1, Spalte 10).

Wenn RNS unter dem Gesichtspunkt des Auftretens des 538 bp intronfreien AT1R-Fragmentes untersucht wurden und diese Reaktionsmischung mit verschiedenen DNase I-Mengen sowie für verschiedene Verdauzeiten (10 Minuten, 20 Minuten, 30 Minuten, 37 °C) untersucht wurden, war zu erkennen, daß je mehr DNase I benutzt wurde und je länger die Inkubationszeit war, desto schwächer das AT1R-spezifische Signal nach reverser Transkriptasereaktion und PCR wurde, obwohl die spezifischen internen Kontrollsignale für die kurzen 103 bp PDH-Fragmente in unveränderter Signalstärke erkennbar waren.

Um zu zeigen, ob das DNS-Signal, welches in der reverse Transkriptase/PCR-Reaktion nach DNase I/Mg²⁺-Behandlung auf die enzymatischen Eigenschaften der MMLV reverse Transkriptase oder die Taq-Polymerase zurückzuführen war, wurden alle Experimente wiederholt, jedoch der reverse Transkriptaseschritt ohne MMLV-Enzym durchgeführt. Unter diesen Bedingungen wurde kein zusätzliches Fragment in Größe der DNS in der (pseudo)-reverse Transkriptase/PCR sichtbar, wenn der DNase I-Verdau in Gegenwart von Mg²⁺-Ionen durchgeführt wurde.

Diese Versuche zeigen, daß nur bei Einsatz von Mn²⁺ als Kofaktor eine Differenzierung des Signals nach reverser Transkriptasereaktion und PCR durchgeführt werden kann, ob das Signal auf (durch reverse Transkriptasereaktion aus RNS gebildeter) cDNS oder Artefakt-DNS zurückzuführen ist. Eine quantitative Aussage über die Anzahl von mRNS-Kopien eines gegebenen Transkripts ist mit Mg²⁺-haltigen Puffern nicht oder nur schwierig möglich. Außerdem können Transkripte seltener mRNS durch Intron enthaltende Gene kompetitiert werden und somit falsch negative Ergebnisse liefern. Andererseits liefern bei intronlosen Genen reassoziierte DNS-Fragmente falsch positive Signale für mRNS-Kopien, die gar nicht transkribiert wurden.

## Patentansprüche

1. Verfahren zur Transkription von Ribonukleinsäuren einer Probe zu Desoxyribonukleinsäuren, enthaltend die Schritte:
a) Behandlung der ribonukleinsäurehaltigen Probe mit einer Lösung von DNAse I und anschließend
b) Transkription der Ribonukleinsäuren in Desoxyribonukleinsäuren,
**dadurch gekennzeichnet, daß** Schritt a) in Anwesenheit von Mn²⁺-Ionen durchgeführt wird.

2. Verfahren zur Herstellung einer Vielzahl von DNS-Molekülen unter Verwendung von RNS-Molekülen, enthaltend die Schritte
a) Behandlung der ribonukleinsäurehaltigen Probe mit einer Lösung von DNAse I und anschließend
b) Transkription der Ribonukleinsäuren in Desoxyribonukleinsäuren und
c) Vermehrung der DNS-Moleküle,
**dadurch gekennzeichnet, daß** Schritt a) in Anwesenheit von Mn²⁺-Ionen durchgeführt wird.

3. Verfahren gemäß Anspruch 2, **dadurch gekennzeichnet, daß** die Vermehrung der DNS-Moleküle mit Hilfe der Polymerasekettenreaktion geschieht.

4. Verfahren gemäß einem der vorangehenden Ansprüche, **dadurch gekennzeichnet, daß** die Endkonzentration von Mn²⁺ während Schritt a) zwischen 0.01 und 5 mM liegt.

5. Verfahren gemäß einem der vorangehenden Ansprüche, **dadurch gekennzeichnet, daß** die Transkription mit Hilfe eines Enzyms mit reverser Transkriptaseaktivität durchgeführt wird.

6. Verfahren gemäß Anspruch 1 oder 2, **dadurch gekennzeichnet, daß** die DNase I vor Schritt b) inaktiviert wird.

7. Reagenz zur Reduzierung der Bildung von Artefakt-DNS bei Transkriptionsreaktionen, **dadurch gekennzeichnet, daß** es eine Lösung von DNase I und Mn²⁺-Ionen enthält.

8. Reagenzkit zur Transkription von Ribonukleinsäuren in Desoxyribonukleinsäuren, enthaltend in getrennten Behältern:
- eine Lösung von DNase I und Mn²⁺-Ionen,
- eine reverse Transkriptase.

9. Reagenzkit zur Herstellung einer Vielzahl von DNS-Molekülen aus RNS-Molekülen, enthaltend in getrennten Behältern:
- eine Lösung von DNase I und Mn²⁺-Ionen,
- eine reverse Transkriptase,
- Reagenzien zur Vervielfältigung von DNS.

10. Verwendung einer Lösung von DNase I und Mn²⁺-Ionen zur Reduktion der Bildung von Artefakt-DNS bei Transkriptionsreaktionen von Ribonukleinsäuren in Desoxyribonukleinsäuren.

## Claims

1. Method for transcribing ribonucleic acids of a sample into deoxyribonucleic acids comprising the steps:
a) treating the sample containing ribonucleic acids with a solution of DNAse I and subsequently
b) transcribing the ribonucleic acids into deoxyribonucleic acids,
wherein step a) is carried out in the presence of Mn²⁺ ions.

2. Method for preparing a plurality of DNA molecules using RNA molecules comprising the steps:
a) treating the sample containing ribonucleic acids with a solution of DNAse I and subsequently
b) transcribing the ribonucleic acids into deoxyribonucleic acids and
c) amplifying the DNA molecules,
wherein step a) is carried out in the presence of Mn²⁺ ions.

3. Method as claimed in claim 2, wherein the DNA molecules are amplified with the aid of the polymerase chain reaction.

4. Method as claimed in one of the previous claims, wherein the final concentration of Mn²⁺ during step a) is between 0.01 and 5 mM.

5. Method as claimed in one of the previous claims, wherein the transcription is carried out with the aid of an enzyme having reverse transcriptase activity.

6. Method as claimed in claim 1 or 2, wherein the DNase I is inactivated before step b).

7. Reagent for reducing the formation of artefact DNA in transcription reactions, wherein it contains a solution of DNase I and Mn²⁺ ions.

8. Reagent kit for transcribing ribonucleic acids into deoxyribonucleic acids containing the following in separate containers:
- a solution of DNase I and Mn²⁺ ions,
- a reverse transcriptase.

9. Reagent kit for preparing a plurality of DNA molecules from RNA molecules containing the following in separate containers:
- a solution of DNase I and Mn²⁺ ions,
- a reverse transcriptase and
- reagents for amplifying DNA.

10. Use of a solution of DNase I and Mn²⁺ ions to reduce the formation of artefact DNA in reactions in which ribonucleic acids are transcribed into deoxyribonucleic acids.

## Revendications

1. Procédé pour la transcription d'acides ribonucléiques d'un échantillon en acides désoxyribonucléiques, comprenant les étapes consistant à:
a) traiter l'échantillon contenant des acides ribonucléiques avec une solution de DNAse I, puis
b) transcrire les acides ribonucléiques en acides désoxyribonucléiques
**caractérisé en ce qu'**on effectue l'étape a) en présence d'ions Mn²⁺.

2. Procédé pour la préparation d'une multitude de molécules d'ADN en utilisant des molécules d'ARN, comprenant les étapes consistant à:
a) traiter l'échantillon contenant des acides ribonucléiques avec une solution de DNAse I, puis
b) transcrire les acides ribonucléiques en acides désoxyribonucléiques, et
c) multiplier les molécules d'ADN
**caractérisé en ce qu'**on effectue l'étape a) en présence d'ions Mn²⁺.

3. Procédé selon la revendication 2, **caractérisé en ce que** la multiplication des molécules d'ADN a lieu à l'aide de la réaction d'amplification en chaîne par polymérase.

4. Procédé selon l'une quelconque des revendications précédentes, **caractérisé en ce que** la concentration finale des ions Mn²⁺ au cours de l'étape a) se situe entre 0,01 et 5 mM.

5. Procédé selon l'une quelconque des revendications précédentes, **caractérisé en ce qu'**on effectue la transcription à l'aide d'une enzyme possédant une réactivité de transcriptase inverse.

6. Procédé selon la revendication 1 ou 2, **caractérisé en ce qu'**on inactive la DNAse I avant l'étape b).

7. Réactif pour réduire la formation d'ADN artefactuel dans des réactions de transcription, **caractérisé en ce qu'**il contient une solution de DNAse I et d'ions Mn²⁺.

8. Nécessaire de réactifs pour la transcription d'acides ribonucléiques en acides désoxyribonucléiques contenant, dans des récipients séparés:
- une solution de DNAse I et d'ions Mn²⁺,
- une transcriptase inverse.

9. Nécessaire de réactifs pour la préparation d'une multitude de molécules d'ADN à partir de molécules d'ARN contenant, dans des récipients séparés:
- une solution de DNAse I et d'ions Mn²⁺,
- une transcriptase inverse,
- des réactifs pour la multiplication de l'ADN.

10. Utilisation d'une solution de DNAse I et d'ions Mn²⁺ pour la réduction de la formation d'ADN artefactuel dans des réactions de transcription d'acides ribonucléiques en acides désoxyribonucléiques.
